# DEMANDE DE BREVET EUROPEEN

(11) **EP 1 676 564 A1**
(43) Date de publication de la demande: **05.07.2006**
(21) Numéro de dépôt: 05292181.4
(22) Date de dépôt: 17.10.2005
(51) Int. Cl.: A61K 8/39, A61K 8/37, A61Q 1/06

(54) **Composition cosmétique contenant un ester d'alcool alcoxylé et une huile apolaire**

(30) Priorité: 30.12.2004 FR 0453263
(71) Demandeur: L'ORÉAL, 75008 Paris (FR)
(72) Inventeur: Lebre, Caroline, 94320 Thiais (FR); Ricard, Audrey, 75012 Paris (FR)
(74) Mandataire: Chantraine, Sylvie Hélène

(57) **Abrégé**

La présente demande concerne une composition comprenant au moins un ester d'alcool alcoxylé et d'acide carboxylique et au moins une huile apolaire, ladite composition étant apte à former un dépôt ayant une brillance moyenne mesurée à 60° supérieure ou égale à 45 sur 100.

L'ester d'alcool alcoxylé peut être notamment l'Octyldodécyl PPG-3 Myristyl Ether Dimer Dilinoléate.

Cette composition peut être utilisée en tant que produit de soin et/ou de maquillage des matières kératiniques, notamment de la peau, des lèvres et/ou des phanères.

## Description

La présente invention se rapporte à une composition cosmétique contenant un ester alcoxylé, notamment une composition cosmétique de maquillage ou de soin de la peau du visage ou du corps humain, du cuir chevelu, des lèvres ou des phanères, comme les cheveux, les cils, les sourcils ou les ongles.

La composition de l'invention peut en particulier constituer un produit de maquillage des lèvres, du corps ou des phanères pouvant être doté de propriétés de soin. La composition de l'invention peut constituer notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

Certains esters alcoxylés ont déjà été utilisés dans des compositions cosmétiques.

Ainsi, la demande US 2002/0192249 décrit des compositions cosmétiques comprenant un ester d'acide monocarboxylique comprenant de 4 à 24 atomes de carbone, et d'alcool comprenant un groupe polypropoxyle et une chaîne alkyle comportant de 2 à 24 atomes de carbone.
Les préparations peuvent comporter, outre l'ester alcoxylé, de l'huile minérale ou de l'huile de paraffine. Ce document décrit également une composition anhydre contenant cet ester et un agent filmogène, et une composition contenant du PPG-3 Myristyl Ether Néoheptanoate. Plus précisément, le document donne des compositions de rouges à lèvres contenant cet ester polypropoxylé en association avec du polyisobutène hydrogéné ; des compositions de fond de teint en crème en émulsion contenant cet ester ; des compositions de filtres solaires en émulsion contenant du laurate d'hexyle, du palmitate d'octyle, et du palmitate de cétyle en association avec cet ester.

Le document US 5.693.316 divulgue des compositions cosmétiques contenant un ester gras alcoxylé obtenu à partir d'un acide dicarboxylique ayant de 2 à 22 atomes de carbone, en particulier l'acide maléique, et d'un excès stoechiométrique de un ou plusieurs alcools gras polyalcoxylés comportant une chaîne alkyle comprenant de 14 à 22 atomes de carbone et un groupe polyalcoxyle. Les préparations peuvent comporter de l'huile minérale ou de l'huile de paraffine comme deuxième émollient. Ce document décrit également une composition anhydre contenant cet ester et un agent filmogène. L'ester alcoxylé est en particulier le Di-PPG-3 Myristyl maléate.

Le document US 6.476.254 divulgue des compositions cosmétiques contenant un ester d'acide dicarboxylique comprenant de 4 à 12 atomes de carbone et d'alcool gras polyalcoxylé dont la partie non alcoxylée comprend de 8 à 36 atomes de carbones. L'ester peut être le Di-PPG-3 Myristyl adipate. La composition peut être anhydre. Elle peut contenir de l'huile minérale ou de l'huile de paraffine.

La demande WO 2003/013439 a pour objet un ester d'acide dicarboxylique en C3-C21 ou tricarboxylique aliphatique en C4-C22, notamment de C3 à C9, et d'alcool gras polyalcoxylé comprenant un radical alkyle en C6-C30, notamment en C18-C22. Ce document décrit une composition cosmétique qui peut contenir de la vaseline, de l'huile minérale, des esters d'acides carboxylique aliphatiques et d'alcools aliphatiques comprenant de 18 à 40 atomes de carbone, un agent filmogène, ou un alcool gras tel que l'alcool cétylique.

Les documents US 5.302.377, US 5.455.025 et US 5.597.555 divulguent des compositions cosmétiques contenant un ester gras alcoxylé d'acide tricarboxylique, en particulier l'acide citrique, avec un excès stoechiométrique de un ou plusieurs alcools gras polyalcoxylés ayant des propriétés émollientes pour des préparations topiques. Les préparations contiennent une huile minérale comme deuxième émollient. Ce document décrit également l'association de cet ester avec un agent filmogène. L'ester est en particulier le Tri-PPG-3 Myristyl citrate.

Le document WO 2004 052076 décrit des compositions cosmétiques contenant des esters mixtes d'alcools polyalcoxylés et d'alcools monohydriques avec des acides polycarboxyliques, notamment des acides dicarboxyliques. Ces compositions peuvent contenir un deuxième agent émollient tel que l'huile minérale ou la vaseline. Les esters mixtes décrits peuvent être formulés en association avec un composé filmogène.

Il a été trouvé, de façon surprenante, que l'association d'un ester alcoxylé et d'une huile apolaire permet d'obtenir des formules dont la brillance est élevée et dont le confort est équivalent à une formule classique.

La présente invention a donc pour objet une composition cosmétique comprenant au moins un ester d'alcool alcoxylé et d'acide carboxylique, et au moins une huile apolaire. Ladite composition est avantageusement apte à former un dépôt dont la brillance moyenne mesurée à 60° est supérieure ou égale à 45 sur 100.

L'invention a également pour objet un procédé cosmétique pour conférer à un film de composition cosmétique des propriétés de brillance et de confort, consistant à introduire dans ladite composition au moins un ester d'alcool alcoxylé et d'acide carboxylique et au moins une huile apolaire.

L'invention a encore pour objet l'utilisation cosmétique d'une composition décrite précédemment, ainsi que l'utilisation cosmétique de l'association d'au moins un ester d'alcool alcoxylé et d'acide carboxylique et d'au moins une huile apolaire, pour obtenir une composition cosmétique dotée de propriétés de brillance et de confort.

L'invention a encore pour objet un procédé de soin et/ou de maquillage des matières kératiniques qui consiste à appliquer sur les matières kératiniques une composition comprenant au moins un ester d'alcool alcoxylé et d'acide carboxylique et au moins une huile apolaire, ladite composition étant apte à former un dépôt dont la brillance moyenne mesurée à 60° peut être supérieure ou égale à 45.

On entend par alcool alcoxylé, un composé hydrocarboné comprenant au moins une fonction -OH, de préférence une seule, et au moins un groupe 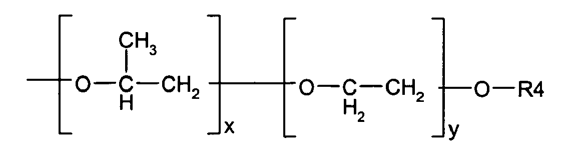 dans laquelle x et y sont indépendamment des entiers de 0 à 40 inclus, et la somme de x et y est comprise entre 1 et 80 inclus,
et R₄ est un motif hydrocarboné aliphatique ou aromatique, saturé ou insaturé, substitué ou non, contenant de 1 à 36 atomes de carbone, notamment de 4 à 36 atomes de carbone.

L'alcool alcoxylé est de préférence un alcool polyalcoxylé tel que le groupe de formule ci-dessus est tel que x et y sont indépendamment des entiers de 0 à 40 inclus, et la somme de x et y est comprise entre 2 et 80 inclus. x et y sont de préférence indépendamment des entiers de 0 à 30 inclus, et la somme de x et y est comprise entre 2 et 30 inclus.

La formule précédente illustre schématiquement tous les motifs éthoxy dans un premier groupe et tous les motifs propoxy dans un autre groupe. En fait, ces motifs peuvent être placés dans n'importe quel ordre, de façon aléatoire, en blocs et sous forme de motifs alternés. A titre purement illustratif, les motifs éthoxy (E) et les motifs propoxy (P) de l'alcool alcoxylé peuvent être disposés de la façon suivante : EEEP, EEPE, EPEE, PEEE, EEEPEPPPE, PEPPPEEEEPE et selon des arrangements similaires.

### ESTER ALCOXYLE

La composition selon l'invention contient au moins un ester d'alcool alcoxylé et d'acide carboxylique, appelé par la suite ester alcoxylé, qui peut être choisi parmi
- les esters obtenus par réaction d'un acide monocarboxylique avec un alcool alcoxylé,
- les polyesters obtenus par réaction d'un acide polycarboxylique avec un excès stoechiométrique d'au moins un alcool alcoxylé par rapport au nombre de fonctions acides dudit acide,
- les polyesters dont une seule fonction ester est obtenue par réaction d'une fonction acide d'un acide polycarboxylique avec un alcool alcoxylé,
- les polyesters comprenant au moins une fonction ester obtenue par réaction d'une fonction acide d'un acide polycarboxylique avec un alcool alcoxylé, et au moins une fonction ester obtenue par réaction d'une autre fonction acide dudit acide polycarboxylique avec un alcool gras,
- et leurs mélanges.

### Ester alcoxylé d'acide monocarboxylique

L'ester alcoxylé peut être choisi parmi les esters d'acide monocarboxylique et d'alcools gras alcoxylé, en particulier polyalcoxylé. En particulier, l'ester alcoxylé est choisi parmi les esters formés par la réaction d'un acide monocarboxylique aliphatique ou aromatique avec un excès stoechiométrique d'un alcool gras polyalcoxylé, par exemple un alcool polypropoxylé.

L'ester alcoxylé d'acide monocarboxylique peut être choisi parmi les monoesters polypropoxylés de formule développée suivante : 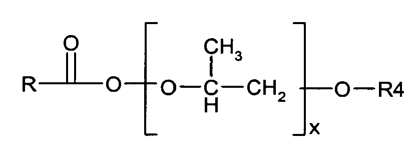 dans laquelle x est un entier de 2 à 40 inclus, de préférence de 3 à 30, notamment entre 3 et 10,
R₄ est un motif hydrocarboné aliphatique saturé ou insaturé, substitué ou non, contenant de 1 à 36 atomes de carbone, de préférence de 3 à 24 atomes de carbone, de préférence de 4 à 24 atomes de carbone et
RCOO correspond à un acide monocarboxylique RCOOH aliphatique ou aromatique.

RCOO peut être
- un reste d'acide monocarboxylique par exemple un acide de formule (R2R3R4C)COO dans laquelle R₂, R₃ et R₄ sont choisis indépendamment dans le groupe constitué de méthyle, d'éthyle, de propyle ou d'isopropyle ; ou
- un reste d'acide aromatique comprenant un cycle benzényle éventuellement substitué par OH ou NH₂ ou méthyle ou éthyle.

Les acides monocarboxyliques aliphatiques convenant à la préparation de l'ester alcoxylé peuvent comprendre de 4 à 24 atomes de carbone, notamment de 4 à 18 atomes de carbone. Des exemples d'acides monocarboxyliques aliphatiques incluent l'acide 2-éthylhexanoïque, l'acide caproïque, l'acide néopentanoïque, l'acide isostéarique, l'acide néoheptanoïque et l'acide oléique.

Des exemples d'acides monocarboxyliques aromatiques incluent l'acide benzoïque et l'acide p-aminobenzoïque.

Les alcools gras utilisés pour préparer les esters alcoxylés peuvent être saturés ou insaturés, substitués ou non, aliphatiques ou aromatiques, et peuvent être à chaîne droite ou à chaîne ramifiée. Ils peuvent avoir entre 6 et 24 atomes de carbone, notamment entre 12 et 14 atomes de carbone.

Par alcool gras, on entend un alcool aliphatique ayant au moins trois atomes de carbone. De préférence, l'alcool gras est constitué d'atomes de carbone, d'hydrogène et d'oxygène. Il peut être saturé ou comporter au moins une double liaison carbone carbone.
Un alcool gras peut notamment être un alcool obtenu par hydrolyse de graisses ou d'huiles végétales ou animales.

Les esters d'acide monocarboxylique et d'alcool gras polypropoxylé sont par exemple choisis parmi le PPG-3 Myristyl Ether Néoheptanoate commercialisé sous la référence Trivasperse, le PPG-4 Butyloctyl Ether Ethylhexanoate et leurs mélanges.

Ces esters peuvent être préparés selon l'enseignement du document US 2002/0192249 dont le contenu est incorporé dans la présente demande par référence.

### Polyesters mixtes alcoxylés

En particulier, l'ester alcoxylé peut être choisi parmi les esters mixtes d'alcool alcoxylé et d'alcool monohydrique avec des acides polycarboxyliques, notamment des acides dicarboxyliques.

Notamment, l'ester alcoxylé est choisi parmi les esters mixtes d'alcool gras polyalcoxylé et d'alcool gras monohydrique avec des acides gras dicarboxyliques.

Par ester mixte on entend un ester obtenu par réaction d'un acide polycarboxylique avec au moins deux alcools différents.

Par acide gras, on entend un acide carboxylique aliphatique ayant au moins trois atomes de carbone. De préférence, l'acide gras est constitué d'atomes de carbone, d'hydrogène et d'oxygène. Il peut être saturé ou comporter au moins une double liaison carbone carbone.
Un acide gras peut notamment être un acide carboxylique obtenu par hydrolyse de graisses ou d'huiles végétales ou animales.

L'ester mixte d'alcool alcoxylé peut être notamment choisi parmi les composés de formule développée suivante : 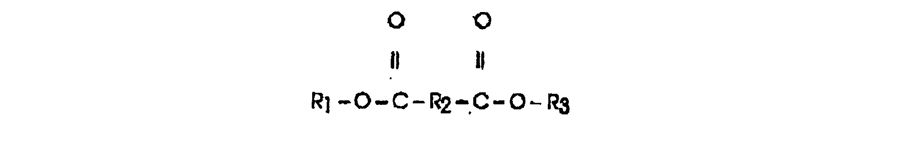 dans laquelle R₁ a la formule développée : 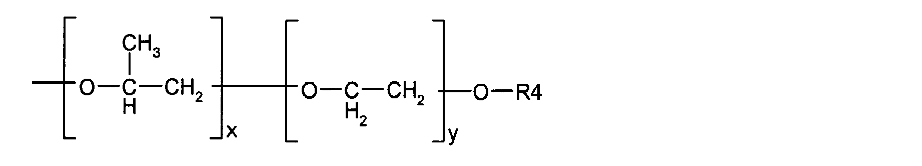 dans laquelle :
R₄ est un motif aliphatique saturé ou insaturé, substitué ou non, contenant de 4 à 24 atomes de carbone ;
x est un entier de 3 à 30 ; y est un entier de 3 à 30
R₂ est un motif aliphatique saturé ou insaturé, substitué ou non, contenant de 4 à 40 atomes de carbone ; et
R₃ est un motif aliphatique à chaîne droite ou à chaîne ramifiée, saturé ou insaturé, contenant de 4 à 32 atomes de carbone, notamment de 12 à 24 atomes de carbone.

Les exemples de composés correspondant à la formule générale précédente sont
- l'Octyldodécyl PPG-3 Myristyl Ether Dimer Dilinoléate commercialisé sous la référence LIQUIWAX polyEFA par la société ARCH CHEMICAL de formule 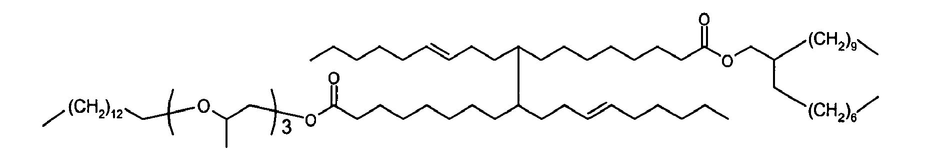
- le Stéaryl PPG-3 Myristyl Ether Dimer Dilinoléate commercialisé sous la référence liquiwax polyIPL par la société ARCH CHEMICAL, et
- l'Isostéaryl PPG-4 Butyloctyl Ether Dimer Dilinoléate.

Ces esters mixtes peuvent être produits par la réaction d'alcools gras alcoxylés et d'alcools gras monohydriques avec des acides gras dicarboxyliques.

De préférence, les alcools gras alcoxylés sont des alcools gras propoxylés et ont une longueur de chaîne carbonée comprise entre 4 et 24 atomes de carbone et un taux de propoxylation compris entre 3 et 30, et de façon tout à fait préférable compris entre 3 et 15 motifs d'oxyde de propylène. Les alcools gras propoxylés préférés sont l'alcool myristique et le butyloctanol.

L'acide dicarboxylique contient au moins deux groupes carboxyliques par molécule. Il peut notamment être représenté par la formule (I) suivante :

HOOC-(CH₂)ₙ-COOH (I)

dans laquelle n est un nombre entier de 1 à 16, de préférence de 3 à 16.
A titre illustratif et non limitatif des acides dicarboxyliques convenant à l'invention, on peut notamment citer l'acide malonique, l'acide succinique, l'acide glutarique, l'acide adipique, l'acide pimélique, l'acide subérique, l'acide azélaïque, l'acide sébacique, l'acide 1,9-nonaméthylène-dicarboxylique, l'acide 1,10-décaméthylènedicarboxylique, l'acide 1,11-ndécaméthylènedicarboxylique, l'acide 1,12-dodécaméthylène-dicarboxylique, l'acide 1,13-tridécaméthylènedicarboxylique, l'acide 1,14-tétradécaméthylènedicarboxylique, l'acide 1,15-pentadécaméthylènedicarboxylique, l'acide 1,16-hexadécaméthylènedicarboxylique et leurs mélanges.

L'acide dicarboxylique peut également être un dimère diacide. Un dimère diacide désigne un diacide obtenu par réaction de polymérisation, notamment de dimérisation, intermoléculaire d'au moins un acide mono-carboxylique insaturé.
Ils dérivent en particulier de la dimérisation d'un acide gras insaturé notamment en C₈ à C₃₄, notamment en C₁₂ à C₂₂, en particulier en C₁₆ à C₂₀, et plus particulièrement en C₁₈.
A titre représentatif de ces acides gras insaturés, on peut notamment citer comme précédemment l'acide undécénoïque, l'acide lindérique, l'acide myristoléique, l'acide palmitoléique, l'acide oléique, l'acide linoléique, l'acide élaïdinique, l'acide gadoléndique, l'acide eicosapentaénoïque, l'acide docosahexaénoïque, l'acide érucique, l'acide brassidique, l'acide arachidonique et leurs mélanges.
Selon une variante particulière, il s'agit plus particulièrement du dimère diacide dont dérive également le dimère diol à estérifier.
Plus particulièrement, il s'agit du dimère diacide obtenu par dimérisation de l'acide linoléique, éventuellement suivie d'une hydrogénation des liaisons carbone carbone. Le dimère diacide peut être sous forme saturée, c'est-à-dire ne comporter aucune double liaison carbone carbone. Selon un autre mode de réalisation, les éventuelles doubles liaisons carbone carbone du dimère diacide sont toutes ou en partie hydrogénées, après réaction d'estérification du dimère diacide avec le dimère diol.
Selon un mode de réalisation, le dimère diacide est un produit commercialisé constitué d'un acide dicarboxylique ayant environ 36 atomes de carbone. Ce produit contient également un acide trimérique et un acide monomère, dans des proportions qui dépendent du degré de pureté du produit. Classiquement, on trouve dans le commerce des produits dont la teneur en dimère diacide est supérieure à 70 % et d'autres dont la teneur en dimère diacide a été ajustée à 90 % ou plus.
On trouve également dans le commerce des dimères diacides et notamment des diacides dilinoléiques dont la stabilité vis-à-vis de l'oxydation a été améliorée par hydrogénation des doubles liaisons restant après la réaction de dimérisation.
Dans la présente invention, on peut utiliser tout dimère diacide disponible actuellement dans le commerce.

Les alcools gras monohydriques peuvent être avoir une longueur de chaîne carbonée comprise entre 12 et 24. Les alcools gras monohydriques préférés sont l'octyldodécanol et l'alcool isostéarique.

Des exemples de préparation des esters décrits précédemment sont donnés dans la demande WO 2004 052076 dont le contenu est incorporé par référence dans la présente demande.

### Polyesters alcoxylés

L'ester alcoxylé peut être obtenu par estérification d'un acide polycarboxylique par au moins deux alcools alcoxylés, lesquels peuvent être identique ou différents, de manière à former un ester.

L'ester peut notamment être choisi parmi les esters de formule : 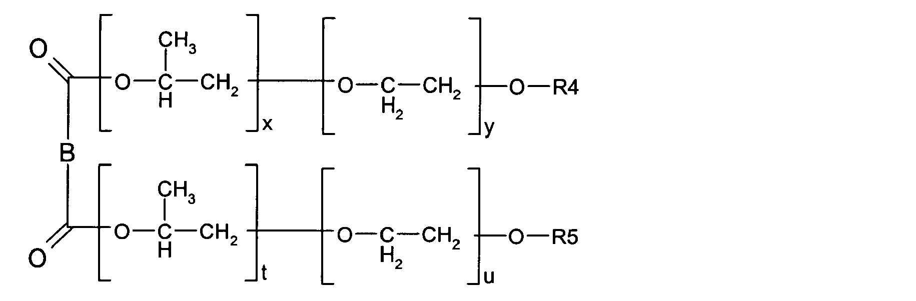 dans laquelle
-OOC-B-COO- est le reste d'un acide dicarboxylique tel que décrit précédemment, ayant notamment de 2 à 40 atomes de carbone, saturé ou insaturé, substitué ou non substitué,
x et y sont indépendamment des entiers de 0 à 40 inclus, et la somme de x et y est comprise entre 1 et 80 inclus, de préférence entre 2 et 80 inclus,
t et u sont indépendamment des entiers de 0 à 40 inclus, et la somme de t et u est comprise entre 1 et 80 inclus, de préférence entre 2 et 80 inclus,
R₄ et R₅ sont indépendamment l'un de l'autre des motifs hydrocarbonés aliphatiques ou aromatiques, saturés ou insaturés, substitués ou non, contenant de 4 à 36 atomes de carbone.

Selon un mode de mise en oeuvre, R₄ et R₅ peuvent être identiques ou différents et comportent notamment de 10 à 22 atomes de carbone et peuvent être saturés ou insaturés, substitués ou non substitués,

Selon un mode de mise en oeuvre, y vaut de 1 à 40 et x vaut de 0 à 30 à la condition que si x est égal à 0, y soit égal à au moins 2 et à la condition supplémentaire que y soit plus grand que x.

Selon un mode de mise en oeuvre, u vaut de 1 à 40 et t vaut de 0 à 30 à la condition que si t est égal à 0, u soit égal à au moins 2 et à la condition supplémentaire que u soit plus grand que t.

L'acide dicarboxylique peut être aliphatique et contenir de 2 à 36 atomes de carbone. Les acides dicarboxyliques aromatiques contiennent avantageusement de 8 à 36 atomes de carbone. Les acides dicarboxyliques aliphatiques préférés contiennent de 3 à 8 atomes de carbone. Les exemples d'acides dicarboxyliques aliphatiques appropriés comprennent l'acide adipique, l'acide sébacique, l'acide malonique, l'acide succinique et l'acide maléique.

Les acides dicarboxyliques aromatiques préférés contiennent de 8 à 12 atomes de carbone. Un exemple d'acide dicarboxylique aromatique approprié est l'acide phtalique. L'acide 1,2-phtalique, qui possède le point de fusion le plus bas des isomères de l'acide phtalique, est préféré.
De préférence, x et y peuvent chacun être égaux à 15, le total de x et y n'excédant pas 25. De préférence, u et t peuvent chacun être égaux à 15, le total de u et t n'excédant pas 25.

Dans un mode de réalisation, y ou u est supérieur ou égal à 1, et x ou t est supérieur ou égal à 0. Le nombre de motifs éthoxy peut être plus grand que le nombre de motifs propoxy.

Les esters alcoxylés peuvent être préparés selon l'enseignement du document WO 00/19972 dont le contenu est incorporé dans la présente demande par référence.

Les groupes aliphatiques saturés non substitués sont préférés pour les diesters d'acides dicarboxyliques de la présente invention, et de tels groupes gras contenant de 14 à 18 atomes de carbone sont davantage préférés. Encore plus préférés sont les groupes contenant de 14 à 16 atomes de carbone. Comme cela est mentionné précédemment, le groupe gras myristyle contenant 14 atomes de carbone est particulièrement préféré.

Dans un mode de réalisation préféré, lorsque R4 et R5 est un groupe myristyle, y et u est de préférence égal à zéro et chaque x et t est de préférence un entier choisi indépendamment parmi 2 à 40 inclus. Un exemple particulièrement préféré est le produit commercialisé sous la référence Cromollient DP3A dans lequel, dans la formule précédente, R₄ et R₅ sont un groupe myristyle, -OOC-B-COO- est un adipate, y=u est égal à 0 et x=t est égal à 3.

L'ester alcoxylé est avantageusement présent dans la composition à raison de 1 à 99 % en particulier, notamment de 2 à 60 % en poids, en particulier de 5 à 40 % et plus particulièrement de 10 à 35 % en poids par rapport au poids total de la composition.

### Huile apolaire

La composition selon l'invention contient au moins une huile apolaire. Par « huile apolaire » au sens de le présente invention, on entend une huile dont le paramètre de solubilité à 25°C δₐ est égal à 0 (J/cm³)^{½}.

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).
Selon cet espace de Hansen :
- δ_{D} caractérise les forces de dispersion de LONDON issues de la formation de dipôles induits lors des chocs moléculaires ;
- δ_{P} caractérise les forces d'interactions de DEBYE entre dipôles permanents ainsi que les forces d'interactions de KEESOM entre dipôles induits et dipôles permanents;
- δₕ caractérise les forces d'interactions spécifiques (type liaisons hydrogène, acide/base, donneur/accepteur, etc.) ;
- δₐ est déterminé par l'équation : δₐ = (δₚ2 + δₕ²)^{½}
Les paramètres δₚ, δₕ, δ_{D} et δₐ sont exprimés en (J/cm³)^{½}.

L'huile apolaire peut être hydrocarbonée.
Par « huile hydrocarbonée », on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.
Selon un mode de réalisation l'huile hydrocarbonée apolaire est dépourvue d'hétéroatome(s). On entend par hétéroatome, un atome différent du carbone ou de l'hydrogène.

L'huile apolaire est de préférence non volatile.
On entend par « huile non volatile », toute huile ayant une pression de vapeur à température ambiante et pression atmosphérique, non nulle inférieure à 0,02 mm de Hg et mieux inférieur à 10⁻³ mm de Hg.

L'huile apolaire représente avantageusement 1% à 80% en poids du poids total de la composition, de préférence de 3% à 50% en poids, de préférence encore de 5% à 30% en poids par rapport au poids total de la composition.

L'huile apolaire représente de préférence de 10% à 40% en poids, de préférence de 15 à 30% en poids par rapport au poids total de la composition.

Selon un mode de réalisation, l'huile apolaire est une huile apolaire hydrocarbonée non volatile qui peut être avantageusement choisie parmi les alcanes saturés, linéaires ou ramifiés.

L'huile apolaire peut être choisie parmi les huiles dont la masse moléculaire est comprise entre 300 et 900 g/mol, de préférence entre 350 et 800 g/mol.

Comme exemples d'huiles apolaires, on peut citer :
- les huiles hydrocarbonées comme le squalène, les hydrocarbures linéaires ou ramifiés tels que les huiles de paraffine, de vaseline et de naphtalène, le polyisobutène hydrogéné ou partiellement hydrogéné, l'isoeicosane, le squalane, les copolymères décène/butène, les copolymères polybutène/polyisobutène notamment l'Indopol L-14, les polydécènes tel que le PURESYN 10, et leurs mélanges;
   Par hydrocarbure, on entend un composé constitué de carbone et d'hydrogène.
- les polydiméthylsiloxanes (PDMS), comportant éventuellement une chaîne alkyle en C₃-C₄₀, ou alcoxy en C₃-C₄₀, ou un radical phénylé ; les polydiméthylsiloxanes comportant des radicaux phénylés peuvent être choisies parmi les phényltriméthicones ;
- les polyalkylméthylsiloxanes, éventuellement fluorées comme les polyméthyltrifluoro-propyldiméthylsiloxanes,
- les polyalkylméthylsiloxanes substituées par des groupements fonctionnels tels que des groupements hydroxyle, thiol et/ou amine;
- les polysiloxanes modifiés par des acides gras, des alcools gras ou des polyoxyalkylènes,
- leurs mélanges.

La composition de l'invention peut contenir plusieurs huiles apolaires. Selon un mode de réalisation, le rapport massique entre ledit au moins ester d'alcool alcoxylé et ladite au moins huile apolaire est compris entre 20/80 et 80/20, notamment entre 30/70 et 70/30, par exemple entre 40/60 et 60/40, notamment de l'ordre de 50/50.

### BRILLANCE

Avantageusement, la composition est apte à former un dépôt ayant une brillance moyenne mesurée à 60° supérieure ou égale à 45. L'huile apolaire est avantageusement telle que lorsqu'elle est en quantité suffisante la composition est apte à former un dépôt ayant une brillance moyenne mesurée à 60° supérieure ou égale à 50.

Par « brillance moyenne », on désigne la brillance telle qu'elle peut être mesurée à l'aide d'un brillancemètre, de manière conventionnelle par la méthode suivante.

Sur une carte de contraste de marque BYK Gardner et de référence Prüfkarten, Art. 2853, prélablement fixée sur une plaque de verre 1 mm, on étale une couche de 25 µm d'épaisseur de la composition à l'aide d'un étaleur automatique (Bar coater, Sheen). La couche recouvre au moins le fond noir de la carte. Lorsque la composition est solide, on la fait fondre si nécessaire sur la carte après l'avoir étalée afin qu'elle recouvre le fond noir. Dès que la composition est étalée, on procède à la mesure de la brillance, dite brillance moyenne T0h, à 60° sur le fond noir à l'aide d'un brillancemètre de marque BYK GARDNER et de référence microTRI-GLOSS. On prépare ainsi quatre cartes de contraste pour mesurer la brillance moyenne de la composition, et on procède à la moyenne des quatre mesures. Pour que la mesure soit correcte, l'écart type doit être inférieur ou égal à 3%.
On laisse ensuite la carte de contraste 5 heures sur une plaque thermostatée à une température de 30°C. Au bout de 5 heures, on retire la carte de contraste de la plaque thermostatée pour qu'elle revienne à la température de la pièce puis on procède à nouveau à la mesure de la brillance moyenne, dite brillance moyenne T5h, comme précédemment.

De préférence, la brillance moyenne de la composition T0h, une fois étalée sur un support, mesurée à 60° est supérieure ou égale à 45, mieux encore, supérieure ou égale à 50, mieux encore, supérieure ou égale à 60, mieux encore, supérieure ou égale à 65, mieux encore, supérieure ou égale à 70, mieux encore, supérieure ou égale à 75.

De préférence, la brillance moyenne de la composition T5h, une fois étalée sur un support, mesurée à 60° est supérieure ou égale à 35, mieux encore, supérieure ou égale à 40, mieux encore, supérieure ou égale à 45, mieux encore, supérieure ou égale à 50, mieux encore, supérieure ou égale à 55, mieux encore, supérieure ou égale à 60, mieux encore, supérieure ou égale à 65, mieux encore, supérieure ou égale à 70 ou mieux encore, supérieure ou égale à 75 sur 100.

Selon un mode de mise en oeuvre, la composition présente avantageusement une bonne tenue de la brillance dans le temps. En particulier, la perte de brillance moyenne au cours du temps est inférieure ou égale à 25%, de préférence inférieure ou égale à 20%, mieux encore inférieure ou égale à 15%, mieux encore inférieure ou égale à 10%, mieux encore inférieure ou égale à 5%.

La perte de la brillance peut notamment être exprimée comme le rapport (T0h-T5h)/T0h.

Selon un mode de mise en oeuvre, la brillance moyenne T0h est supérieure ou égale à 70 sur 100, et la brillance moyenne T5h est supérieure ou égale à 70 sur 100. Selon un autre mode de mise en oeuvre, la brillance moyenne T0h est supérieure ou égale à 75 sur 100, et la brillance moyenne T5h est supérieure ou égale à 75 sur 100.

### PATEUX

La composition peut également contenir en outre au moins un composé pâteux.

La composition est avantageusement exempte de lanoline ou de dérivés de lanoline.

Ainsi, un objet de la présente invention est un composition cosmétique comprenant au moins un ester d'alcool alcoxylé et d'acide carboxylique et au moins une huile apolaire, ladite composition étant apte à former un dépôt ayant une brillance moyenne mesurée à 60° supérieure ou égale à 30 sur 100, et ladite composition étant exempte de lanoline ou d'un de ses dérives.
Dans ce mode de mise en oeuvre, la brillance est avantageusement mesurée selon la méthode décrite précédemment. Elle peut être supérieure ou égale à 35 sur 100, voir même supérieure ou égale à 40 sur 100.

A titre illustratif des dérivés de lanoline utilisés conventionnellement, on peut notamment citer la lanoline liquide, la lanoline réduite, la lanoline purifiée par adsorption, la lanoline acétylée, la cire de lanoline oxypropylénée (5 OP), l'acétate de lanoline liquide, l'hydroxylanoline, la polyoxyéthylène-lanoline, l'acide gras de lanoline, l'acide gras de lanoline dure, les esters de cholestéryle d'acide gras de lanoline, l'alcool de lanoline, l'acétate de l'alcool de lanoline, le lanolate d'isopropyle et autres.

Les lanolines présentent l'inconvénient d'être sensibles à la chaleur et aux ultraviolets. Elles ont tendance à s'oxyder avec un dégagement d'odeur désagréable, et leur couleur très jaune empêche de les utiliser dans des bases de soin non pigmentées et les bases incolores, ce qui limite leur utilisation dans les compositions cosmétiques.

Le demandeur a découvert que les esters alcoxylés décrits précédemment sont de bons substituts de la lanoline ou de ses dérivés.

Par "pâteux" au sens de la présente invention, on entend désigner un composé gras lipophile, à changement d'état solide/liquide réversible, et comportant à la température de 23°C une fraction liquide et une fraction solide. On entend également par « pâteux », le polylaurate de vinyle.

Le composé pâteux peut être choisi parmi :
- la lanoline et ses dérivés,
- les composés fluorés polymères ou non,
- les composés siliconés polymères ou non,
- les polymères vinyliques, notamment:
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈₋C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
   - les polyéthers liposolubles résultant de la polyéthérification entre un ou plusieurs diols en C₂-C₁₀₀, de préférence en C₂-C₅₀,
   - les esters,
      et leurs mélanges.

Parmi les polyéthers liposolubles, on préfère en particulier les copolymères d'éthylène-oxyde et/ou de propylène-oxyde avec des alkylènes-oxydes à longue chaîne en C₆-C₃₀, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les pâteux esters, on préfère notamment :
- les esters d'un glycérol oligomère, notamment les esters de diglycérol, en particulier les condensats d'acide adipique et de glycérol, pour lesquels une partie des groupes hydroxyle des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en C₄-C₅₀ et un diol ou un polyol en C₂-C₅₀, différent du polyester décrit précédemment,
- les esters aliphatiques d'ester résultant de l'estérification d'un ester d'acide hydroxycarboxylique aliphatique par un acide monocarboxylique aliphatique; et leurs mélanges, comme
   - l'ester résultant de la réaction d'estérification de l'huile de ricin hydrogénée avec l'acide isostéarique dans les proportions 1 pour 1 (1/1) ou monoisostéarate d'huile de ricin hydrogénée,
   - l'ester résultant de la réaction d'estérification de l'huile de ricin hydrogénée avec l'acide isostéarique dans les proportions 1 pour 2 (1/2) ou le diisostéarate d'huile de ricin hydrogénée,
   - l'ester résultant de la réaction d'estérification de l'huile de ricin hydrogénée avec l'acide isostéarique dans les proportions 1 pour 3 (1/3) ou triisostéarate d'huile de ricin hydrogénée,
   - et leurs mélanges.

Parmi les composés pâteux d'origine végétale, on choisira de préférence un mélange de stérols de soja et de pentaérythritol oxyéthyléné (50E) oxypropyléné (5 OP), commercialisé sous la référence Lanolide par la société VEVY.

Le composé pâteux représente de préférence 1 à 99%, mieux 1 à 60%, mieux 2 à 30% et mieux encore 5 à 15% en poids de la composition.

### COLORANT

Avantageusement, la composition de l'invention peut comprendre, en outre, au moins une matière colorante qui peut être choisie parmi les colorants, les pigments, les nacres et leurs mélanges. Cette matière colorante peut représenter de 0,001 à 98 %, de préférence de 0,5 à 85% et mieux de 1 à 60 % du poids total de la composition.

Les colorants sont de préférence des colorants liposolubles, bien que les colorants hydrosolubles puissent être utilisés. Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

Pour une composition sous forme de pâte ou coulée telle que les rouges à lèvres ou les produits de maquillage du corps, on utilise en général de 0,5 à 50% de matière colorante, de préférence de 2 à 40 % et mieux de 5 à 30%, par rapport au poids total de la composition.

Par pigments, il faut comprendre des particules blanches ou colorées, minérales ou organiques, insolubles dans la ou les huiles de la composition, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.
La composition contient avantageusement des pigments goniochromatiques, par exemple des pigments multicouches interférentiels, et/ou des pigments réfléchissants. Ces deux types de pigments sont décrits dans la demande FR0209246 dont le contenu est incorporé par référence dans la présente demande.

### CHARGES

La composition peut contenir au moins une charge qui peut être présente à raison de 0,001 à 35 % du poids total de la composition, de préférence 0,5 à 15 %.

On peut notamment citer
- le talc, le mica, le kaolin, l'amidon
- les poudres de Nylon® (Orgasol notamment)
- les poudres de polyéthylène,
- les poudres de polytétrafluoroéthylène (Téflon®),
- le nitrure de bore,
- des microsphères de copolymères telles que l'Expancel® (Nobel Industrie),
- le Polytrap® 603 (Dow Corning),
- le Polypore® L 200 (Chemdal Corporation),
- les microbilles de résine de silicone (Tospearl ® de Toshiba, par exemple),
- les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass
- les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI,
- les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle ; par exemple la lauroyl lysine.

La charge peut être par exemple une charge dont la granulométrie moyenne est inférieure à 100 µm, notamment comprise entre 1 et 50 µm, par exemple entre 4 et 20 µm.

La charge peut être de toute forme, essentiellement sphérique ou sous la forme de plaquettes.

### CIRE

La composition peut contenir, en outre, au moins une cire. Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.
Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.

La composition selon l'invention contient avantageusement de la cire de polyéthylène de masse moléculaire en poids comprise entre 300 et 700, notamment égale à 500 g/mol.

A titre indicatif, la cire peut représenter de 0,01 à 50 %, de préférence de 2 à 40 %, et mieux de 5 à 30 % du poids total de la composition.

### HUILE NON VOLATILE

La composition peut également comprendre en outre au moins une huile non volatile, autre que l'ester d'alcool alcoxylé et autre que l'huile apolaire décrits précédemment. L'huile non volatile peut être choisie parmi:
- les huiles hydrocarbonées végétales telles que les triglycérides liquides d'acides gras ayant de 4 à 10 atomes de carbone comme les triglycérides des acides heptanoïque ou octanoïque ou l'huile de jojoba ;
- les esters hydrocarbonés de formule RCOOR' dans laquelle RCOO représente un reste d'acide carboxylique comportant de 2 à 30 atomes de carbone, et R' représente une chaîne hydrocarbonée contenant de 1 à 30 atomes de carbone, tel que l'isononanoate d'isononyle, l'érucate d'oléyle ou le néopentanoate d'octyl-2-docécyle ;
- les alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les acides gras ayant de 12 à 26 atomes de carbone comme l'acide oléïque ;
- et leurs mélanges.

### Huile non volatile de masse moléculaire élevée

Selon un mode de réalisation, la composition contient une huile non volatile de masse moléculaire élevée, par exemple comprise entre 650 à 10000 g/mol.

La composition selon l'invention contient avantageusement de 2 à 30 %, de préférence de 5 à 25%, de 5 à 15% d'au moins une huile de masse molaire allant de 650 à 10000 g/mol, et de préférence allant de 900 et 7500 g/mol.

L'huile non volatile de masse moléculaire élevée peut être une huile apolaire telle que décrite précédemment, sachant que selon un mode de réalisation de l'invention, l'huile apolaire de la composition a une masse moléculaire comprise entre 300 et 900 g/mol.

Ainsi, l'huile de masse moléculaire allant de 650 à 10000 g/mol peut être choisie parmi
- les polybutylènes tels que L'INDOPOL H-100 (de masse molaire ou MM=965 g/mol), L'INDOPOL H-300 (MM=1340 g/mol), L'INDOPOL H-1500 (MM=2160g/mol) commercialisés ou fabriqués par la société AMOCO,
- les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
- les polydécènes et les polydécènes hydrogénés tels que le PURESYN 150 (MM=9200 g/mol) commercialisé par la société MOBIL CHEMICALS,
- les copolymères de la vinylpyrrolidone tels que le copolymère vinylpyrrolidone/1-héxadécène, ANTARON V-216 commercialisé ou fabriqué par la société ISP (MM=7300 g/mol),
- les esters tels que :
   a) les esters d'acides gras linéaires ayant un nombre total de carbone allant de 35 à 70 comme le tétrapélargonate de pentaérythrityle (MM=697,05 g/mol),
   b) les esters hydroxylés tels que le triisostéarate de polyglycérol-2 (MM=965,58 g/mol),
   c) les esters aromatiques tels que le tridécyl trimellitate (MM=757,19 g/mol),
   d) les esters d'alcool gras ou d'acides gras ramifiés en C₂₄-C₂₈ tels que ceux décrits dans la demande EP-A-0 955 039, et notamment le citrate de triisoarachidyle (MM=1033,76 g/mol), le tétraisononanoate de pentaérythrityle (MM=697,05g/mol), le triisostéarate de glycéryle (MM=891.51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
   e) les esters et polyesters de dimère diol, tels que les esters de dimère diol et d'acide gras , et les esters de dimère diols et de diacide.
   Les esters de dimère diol et d'acide mono-carboxylique peuvent être obtenus à partir d'acide mono-carboxylique comprenant de 4 à 34 atomes de carbone, notamment de 10 à 32 atomes de carbone, lesquels acides sont linéaires, ramifiés, saturés ou insaturés.
   A titre illustratif des exemples d'acide mono-carboxylique convenant à l'invention, on peut notamment citer les acides gras.
   Les esters de dimère diol et d'acide dicarboxylique peuvent être obtenus à partir d'un dimère diacide dérivé en particulier de la dimérisation d'un acide gras insaturé notamment en C₈ à C₃₄, notamment en C₁₂ à C₂₂, en particulier en C₁₆ à C₂₀, et plus particulièrement en C₁₈.
   Selon une variante particulière, il s'agit plus particulièrement du dimère diacide dont dérive également le dimère diol à estérifier.
   Les esters de dimère diol peuvent être obtenus à partir d'un dimère diol produit par hydrogénation catalytique d'un dimère diacide tel que décrit précédemment, par exemple le diacide dilinoléique hydrogéné.
   A titre illustratif des esters de dimère diol, on peut notamment citer les esters de diacides dilinoléiques et de dimères diols dilinoléiques commercialisés par la société NIPPON FINE CHEMICAL sous la dénomination commerciale LUSPLAN DD-DA5® et DD-DA7® .
- les huiles siliconées telles que les silicones phénylées comme la BELSIL PDM 1000 de la société WACKER (MM=9000 g/mol),
- les huiles d'origine végétale telles que l'huile de sésame (820,6 g/mol),
- et leurs mélanges.

Les huiles non volatiles peuvent représenter de 0,001 à 90 % du poids total de la composition, de préférence de 0,05 à 60 % et mieux de 1 à 35 %.

### HUILE VOLATILE

On peut inclure une ou plusieurs huiles volatiles dans la composition.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 13 000 Pa (0,01 à 100 mm de Hg), et préférentiellement allant de 1,3 Pa à 1 300 Pa (0,1 à 10 mm de Hg).

En outre, l'huile volatile a généralement un point d'ébullition, mesuré à pression atmosphérique, allant de 150 °C à 260 °C, et de préférence allant de 170 °C à 250 °C.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par huile siliconée, on entend une huile contenant au moins un atome de silicium, et notamment contenant des groupes Si-O.

Par huile fluorée, on entend une huile contenant au moins un atome de fluor.

L'huile volatile peut être siliconée ou hydrocarbonée.

L'huile volatile siliconée utilisable dans l'invention peut être choisie parmi les huiles siliconées ayant un point éclair allant de 40 °C à 102 °C, de préférence ayant un point éclair supérieur à 55 °C et inférieur ou égal à 95 °C, et préférentiellement allant de 65 °C à 95 °C.

Comme huiles siliconées volatiles utilisables dans l'invention, on peut citer les silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl-cyclotétrasiloxane, le décaméthyl-cyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huiles siliconées volatiles utilisables dans l'invention, on peut citer les silicones décrites dans la demande FR0304259 non publiée.

L'huile volatile hydrocarbonée utilisable dans l'invention peut être choisie parmi les huiles hydrocarbonées ayant un point éclair allant de 40 °C à 102 °C, de préférence allant de 40 °C à 55 °C, et préférentiellement allant de 40 °C à 50 °C.

Comme huile volatile hydrocarbonée, on peut citer les huiles volatiles hydrocarbonées ayant de 8 à 16 atomes de carbone et leurs mélanges, et notamment les alcanes ramifiés en C₈-C₁₆ comme les iso-alcanes (appelées aussi isoparaffines) en C₈-C₁₆, l'isododécane, l'isodécane, l'isohexadécane et par exemple les huiles vendues sous les noms commerciaux d'Isopars ou de Permetyls, les esters ramifiés en C₈-C₁₆ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile représente par exemple de 5 à 97,5 % du poids total de la composition, et mieux de 10 à 75 %, de préférence entre 20 et 50% du poids total de la composition.

L'huile volatile représente de préférence 20 à 50 % en poids de la composition, de préférence de 30 à 40%, de préférence 35% environ.

### ADDITIFS

La composition de l'invention peut comprendre, en outre, tout additif complémentaire usuellement utilisé dans le domaine cosmétique, tel que de l'eau, des antioxydants, des polymères filmogènes, des conservateurs, des neutralisants, des plastifiants, des gélifiants lipophiles ou des composés non aqueux liquides, des gélifiants de phase aqueuse, des dispersants, des actifs cosmétiques. Ces additifs, à l'exception de l'eau qui peut représenter de 0 à 70 % et par exemple de 1 à 50 et mieux de 1 à 10 % du poids total de la composition, peuvent être présents dans la composition à raison de 0,0005 à 20% du poids total de la composition et mieux de 0,001 à 10%.

Comme actif cosmétique utilisable dans l'invention, on peut citer les vitamines A, E, C, B₃, F, les provitamines comme le D-panthénol, la glycérine, les actifs apaisants comme l'α-bisabolol, l'aloe vera, l'allantoïne, les extraits de plantes ou les huiles essentielles, les agents protecteurs ou restructurants comme les céramides, les actifs "fraîcheur" comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

### GALENIQUES

Les applications des compositions selon l'invention sont multiples et concernent l'ensemble des produits cosmétiques colorés ou non et plus particulièrement les rouges à lèvres.

La composition de l'invention peut se présenter sous la forme de composition solide, compactée ou coulée notamment en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

Elle peut se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. De préférence, elle se présente sous forme de composition à phase continue huileuse et notamment anhydre ; dans ce cas, elle peut contenir une phase aqueuse à un taux inférieur à 5 %.

La composition selon l'invention peut se présenter sous la forme d'une composition, colorée ou non, de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent), produit de bronzage artificiel de la peau.

La composition de l'invention peut également se présenter sous la forme d'un produit de maquillage coloré de la peau, en particulier du visage comme un blush, un fard à joues ou à paupières, de maquillage du corps comme un produit de tatouage semi-permanent ou de maquillage des lèvres comme un rouge ou un brillant à lèvres, présentant éventuellement des propriétés de soin ou de traitement non thérapeutique, un produit de maquillage des phanères comme par exemple un vernis à ongles, un mascara, un eyeliner, un produit de coloration ou de soin des cheveux.

De préférence, la composition selon l'invention se présente sous forme d'un rouge à lèvres ou d'un brillant à lèvres.

Bien entendu la composition de l'invention doit être physiologiquement acceptable (en particulier cosmétiquement acceptable), à savoir non toxique et susceptible d'être appliquée sur la peau, les phanères ou les lèvres d'êtres humains.
Par « cosmétiquement acceptable », on entend agréable de goût, de toucher, d'aspect et/ou d'odeur, applicable plusieurs jours pendant plusieurs mois.

La composition selon l'invention peut être fabriquée par les procédés connus, généralement utilisés dans le domaine cosmétique.

Les exemples qui suivent ont pour but d'illustrer de manière non limitative l'objet de la présente invention. Les quantités sont données en pourcentage massique.

### Exemple 1 : Stick de rouge à lèvres

| **Nom chimique** | **Quantité en gramme** |
|---|---|
| Ethers de dodécanediol et de polyéthylène glycol | 10.3 |
| Octyldodécyl PPG-3 myristyl ether dimer dilinoléate (LIQUIWAX POLYEFA) | 22.15 |
| Néopentanoate d'isostéaryle | 30.3 |
| Stéaroyl stéarate d'isocétyle | 20.6 |
| Polylaurate de vinyle | 16.5 |
| Ditertiobutyl 4-hydroxytoluène | 0.15 |
| Copolymère acétate de vinyle / stéarate d'allyle | 7.5 |
| Polyisobutène hydrogéné | 10 |
| Copolymère polybutène/polyisobutène | 10 |
| Cire de polyéthylène | 3.4 |
| Cire microcristalline | 2.55 |
| Stéarate d'octacosanyle | 4.25 |
| Esters d'acides et alcools linéaires | 3.4 |
| Oxyde de titane rutile traité alumine/silice / tri-méthylolpropane | 0.27 |
| Laque d'aluminium de sel di-sodique de phloxine B sur alumine, benzoate d'aluminium | 0.66 |
| Sel de calcium du rouge lithol B | 0.16 |
| Oxydes de fer brun, jaune | 0.64 |
| Oxyde de fer noir | 0.66 |
| Microsphères de silice poreuse enrobées de polydiméthylsiloxane | 5 |
| parfum | 0.4 |

La brillance moyenne de ce rouge à lèvres T0h et T5h mesurée à 60°, selon la méthode décrite précédemment, sont égales à 79 sur 100.

### Exemple 2 : Stick de rouge à lèvres

| **NOM CHIMIQUE** | **(% en poids)** |
|---|---|
| octyl dodécanol | 14,7 |
| phenyl triméthicone (DC 556 de Dow Corning, 20 cSt) | 4,21 |
| diisostéaryl malate | 12,88 |
| pentaérythrityl tétraisostearate | 10,31 |
| hydrogenated castor oil isostéarate | 9,49 |
| bis-diglycéryl polyacyladipate-2 | 9,49 |
| stéaryl/PPG-3 myristyl ether dimer dilinoléate (Liquiwax PolylPL) | 9,49 |
| C18-36 acid triglycéride | 0,71 |
| conservateur | 0,06 |
| microcristalline wax (MICROWAX HW de Paramelt) | 3,75 |
| hydrogenated coco-glycerides | 6,25 |
| polyethylene wax (Performalene 500 polyethylene de NPT) | 10 |
| pigments | 8,66 |

La brillance moyenne T0h et T5h de la composition mesurée à 60° selon la méthode décrite précédemment sont égales à 72 sur 100

## Revendications

1. Composition cosmétique comprenant au moins un ester d'alcool alcoxylé et d'acide carboxylique et au moins une huile apolaire, ladite composition étant apte à former un dépôt ayant une brillance moyenne mesurée à 60° supérieure ou égale à 45 sur 100.

2. Composition cosmétique comprenant au moins un ester d'alcool alcoxylé et d'acide carboxylique et au moins une huile apolaire, **caractérisée en ce que** l'ester alcoxylé est choisi parmi les polyesters dont une seule fonction ester est obtenue par réaction d'une fonction acide d'un acide polycarboxylique avec un alcool alcoxylé.

3. Composition selon la revendication 1, **caractérisée en ce que** l'ester est obtenu par réaction d'un acide monocarboxylique avec un alcool polyalcoxylé.

4. Composition selon la revendication précédente, **caractérisée en ce que** l'ester alcoxylé est choisi parmi les composés de formule développée suivante : 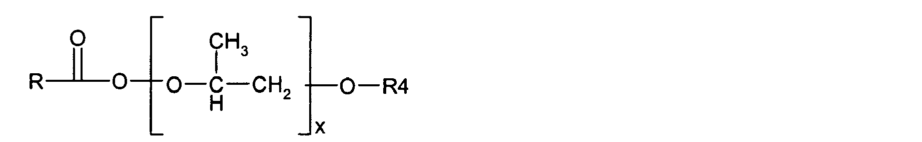 dans laquelle x est un entier de 2 à 40 inclus, de préférence de 3 à 30,
R₄ est un motif hydrocarboné aliphatique saturé ou insaturé, substitué ou non, contenant de 3 à 36 atomes de carbone, de préférence de 4 à 24 atomes de carbone, et
RCOO peut être
- un reste d'acide monocarboxylique par exemple un acide de formule (R2R3R4C)COO dans laquelle R₂, R₃ et R₄ sont choisis indépendamment dans le groupe constitué de méthyle, d'éthyle, de propyle ou d'isopropyle ; ou
- un reste d'acide aromatique comprenant un cycle benzényle éventuellement substitué par OH ou NH₂ ou méthyle ou éthyle.

5. Composition selon la revendication précédente, **caractérisée en ce que** l'acide monocarboxylique est choisi parmi l'acide 2-éthylhexanoïque, l'acide caproïque, l'acide néopentanoïque, l'acide isostéarique, l'acide néoheptanoïque et l'acide oléique.

6. Composition selon l'une des revendications 4 ou 5, **caractérisée en ce que** R₄ comprend entre 4 et 24 atomes de carbone, notamment entre 6 et 18 atomes de carbone.

7. Composition selon l'une des revendications 4 à 6, **caractérisée en ce que** x est compris entre 3 et 10.

8. Composition selon l'une des revendications 3 à 7, **caractérisée en ce que** l'ester d'acide monocarboxylique aliphatique et aromatique d'alcools gras polypropoxylés est choisi parmi le PPG-3 Myristyl Ether Néoheptanoate, le PPG-4 Butyloctyl Ether Ethylhexanoate et leurs mélanges.

9. Composition selon la revendication 1, **caractérisée en ce que** l'ester alcoxylé est choisi parmi les polyesters obtenus par réaction d'un acide polycarboxylique avec un excès stoechiométrique d'au moins un alcool alcoxylé par rapport au nombre de fonctions acides dudit acide.

10. Composition selon la revendication 9, **caractérisée en ce que** l'alcool alcoxylé contient une chaîne alkyle de 8 à 36 atomes de carbone, notamment de 10 à 22 atomes de carbone.

11. Composition selon la revendication 9 ou 10, **caractérisée en ce que** l'alcool alcoxylé contient une chaîne myristyle.

12. Composition selon l'une des revendications 9 à 11, **caractérisée en ce que** l'acide dicarboxylique est aliphatique et contient de 2 à 36 atomes de carbone.

13. Composition selon la revendication 12, **caractérisé en ce que** l'acide dicarboxylique est choisi parmi le groupe constitué des acides adipique, malonique, succinique, phtalique et maléique.

14. Composition selon la revendication 1, **caractérisée en ce que** l'ester alcoxylé est un les polyesters dont une seule fonction ester est obtenue par réaction d'une fonction acide d'un acide polycarboxylique avec un alcool alcoxylé.

15. Composition selon la revendication 1 ou 2, **caractérisée en ce que** l'ester alcoxylé est choisi parmi les composés de formule développée suivante : 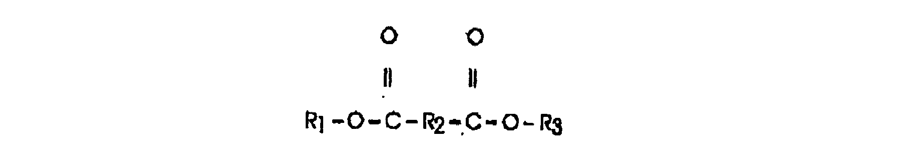 dans laquelle R₁ a la formule développée : 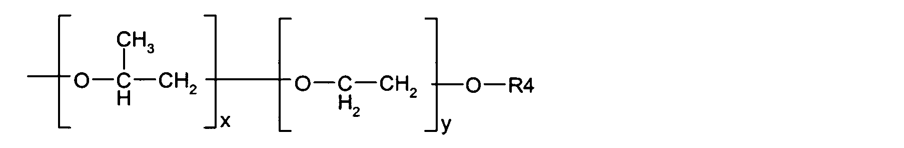 dans laquelle :
R₄ est un motif aliphatique saturé ou insaturé, substitué ou non, contenant de 4 à 24 atomes de carbone ;
x est un entier de 3 à 30 ; y est un entier de 3 à 30
R₂ est un motif aliphatique saturé ou insaturé, substitué ou non, contenant de 4 à 40 atomes de carbone ; et
R₃ est un motif aliphatique à chaîne droite ou à chaîne ramifiée, saturé ou insaturé, contenant de 4 à 32 atomes de carbone, notamment de 12 à 24 atomes de carbone.

16. Composition selon la revendication 15, **caractérisé en ce que** R₄ est un motif aliphatique saturé de 12 ou 20 atomes de carbone.

17. Composition selon la revendication 15 ou 16, **caractérisé en ce que** R₂ est un motif aliphatique saturé contenant de 4 à 40 atomes de carbone.

18. Composition selon la revendication 15, **caractérisé en ce que** x ou y sont indépendamment l'un de l'autre égaux à 3 ou 4.

19. Composition selon la revendication 15, **caractérisé en ce que** R₃ est un motif aliphatique saturé à chaîne ramifiée, contenant de 12 à 20 atomes de carbone.

20. Composition selon la revendication 19, **caractérisé en ce que** R₃ est choisi parmi l'octyldodécyle, l'isostéaryle et le stéaryle.

21. Composition selon la revendication 15, **caractérisé en ce que** l'ester alcoxylé est l'Octyldodécyl PPG-3 Myristyl Ether Dimer Dilinoléate.

22. Composition selon la revendication 15, **caractérisé en ce que** l'ester alcoxylé est l'Isostéaryl PPG-4 Butyloctyl Ether Dimer Dilinoléate.

23. Composition selon l'une des revendications précédentes, caratérisée en ce que l'huile apolaire est telle, que lorsqu'elle est en quantité suffisante dans la composition, ladite composition est apte à former un dépôt dont la brillance moyenne est supérieure ou égale à 45 sur 100.

24. Composition selon l'une quelconque des revendications précédentes, **caractérisée en ce que** l'huile apolaire est non volatile.

25. Composition selon l'une quelconque des revendications précédentes, **caractérisée en ce que** l'huile apolaire est choisie parmi les huiles hydrocarbonées.

26. Composition selon l'une quelconque des revendications précédentes, **caractérisée en ce que** l'huile apolaire est dépourvue d'hétéroatome(s).

27. Composition selon la revendication précédente, **caractérisée en ce que** l'huile apolaire est choisie parmi les hydrocarbures.

28. Composition selon la revendication précédente, **caractérisée en ce que** l'huile hydrocarbonée apolaire est choisie parmi les alcanes saturés, linéaires ou ramifiés.

29. Composition selon l'une quelconque des revendications précédentes, **caractérisée en ce que** l'huile apolaire est choisie parmi les huiles hydrocarbonées apolaires dont la masse moléculaire est comprise entre 300 et 900 g/mol, de préférence entre 350 et 800 g/mol.

30. Composition selon les revendications 24 et 26, **caractérisée en ce que** l'huile apolaire hydrocarbonée non volatile est choisie parmi les hydrocarbures linéaires ou ramifiés tels que les huiles de paraffine, de vaseline et de naphtalène, le polyisobutène hydrogéné, les copolymères polyisobutène/polybutène, l'isoeicosane, le squalane, les polydécènres, les copolymères décène/butène et leurs mélanges.

31. Composition selon l'une des revendications précédentes, **caractérisée en ce que** la brillance moyenne de la composition T0h, une fois étalée sur un support, mesurée à 60° est supérieure ou égale à 45, mieux encore, supérieure ou égale à 50, mieux encore, supérieure ou égale à 60, mieux encore, supérieure ou égale à 65, mieux encore, supérieure ou égale à 70, mieux encore, supérieure ou égale à 75.

32. Composition selon l'une des revendications précédentes, **caractérisée en ce que** la brillance moyenne de la composition T5h, une fois étalée sur un support, mesurée à 60° est supérieure ou égale à 35, mieux encore, supérieure ou égale à 40, mieux encore, supérieure ou égale à 45, mieux encore, supérieure ou égale à 50, mieux encore, supérieure ou égale à 55, mieux encore, supérieure ou égale à 60, mieux encore, supérieure ou égale à 65, mieux encore, supérieure ou égale à 70 ou mieux encore, supérieure ou égale à 75 sur 100.

33. Composition selon l'une des revendications 31 et 32, **caractérisée en ce que** la perte de brillance moyenne au cours du temps exprimée comme le rapport (T0h-T5h)/T0h est inférieure ou égale à 25%, de préférence inférieure ou égale à 20%, mieux encore inférieure ou égale à 15%, mieux encore inférieure ou égale à 10%, mieux encore inférieure ou égale à 5%.

34. Composition selon l'une quelconque des revendications précédentes, **caractérisée en ce qu'**elle comprend, en outre, une ou des matières colorantes choisies parmi les colorants hydrosolubles ou liposolubles et les matières colorantes pulvérulentes, tels que les pigments, les nacres et les paillettes.

35. Composition selon l'une quelconque des revendications précédentes, **caractérisée par le fait qu'**elle comprend en outre au moins un corps gras choisi parmi les cires, les corps gras pâteux et leurs mélanges.

36. Composition selon l'une quelconque des revendications précédentes, **caractérisée par le fait qu'**elle comprend un ingrédient cosmétique choisi parmi les vitamines, les épaississants, les filmogènes, les gélifiants, les oligo-éléments, les adoucissants, les séquestrants, les parfums, les agents alcalinisants ou acidifiants, les conservateurs, les filtres solaires, les tensioactifs, les antioxydants, les fibres, les agents anti-chutes des cheveux, les agents de soin du cil, les agents anti-pelliculaires, les agents propulseurs, ou leurs mélanges.

37. Composition cosmétique selon l'une quelconque des revendications précédentes, **caractérisée en ce qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre, de stick ou de solide coulé.

38. Composition cosmétique selon l'une quelconque des revendications 1 à 33, **caractérisée en ce qu'**elle se présente sous forme anhydre.

39. Composition cosmétique comprenant au moins un ester d'alcool alcoxylé et d'acide carboxylique et au moins une huile apolaire, ladite composition étant apte à former un dépôt ayant une brillance moyenne mesurée à 60° supérieure ou égale à 30 sur 100, et ladite composition étant exempte de lanoline ou d'un de ses dérives.

40. Composition cosmétique selon l'une quelconque des revendications précédentes, **caractérisée en ce qu'**il s'agit d'une composition de maquillage ou de soin des matières kératiniques.

41. Composition selon l'une des revendications 1 à 39, **caractérisée en ce qu'**il s'agit d'un produit de maquillage des lèvres.

42. Utilisation cosmétique d'une composition selon l'une des revendications 1 à 41.

43. Procédé de maquillage et/ou de soin de la peau, des lèvres comprenant l'application sur la peau, les lèvres et/ou les phanères d'au moins une composition selon l'une quelconque des revendications 1 à 41.
